Europäisches Patentamt

European Patent Office

Office européen des brevets

(11) Publication number: **0 321 616 B1**

# EUROPEAN PATENT SPECIFICATION

(45) Date of publication of patent specification: **20.04.94**    (51) Int. Cl.5: **C07C 1/06**

(21) Application number: **87311323.7**

(22) Date of filing: **22.12.87**

Divisional application 92202404.7 filed on 22/12/87.

(54) **Process for reducing methane production and increasing liquid yields in Fischer-Tropsch reactions.**

(43) Date of publication of application:
**28.06.89 Bulletin 89/26**

(45) Publication of the grant of the patent:
**20.04.94 Bulletin 94/16**

(84) Designated Contracting States:
**BE DE FR GB IT NL**

(56) References cited:
**US-A- 4 547 525**

(73) Proprietor: **EXXON RESEARCH AND ENGI-
NEERING COMPANY
P.O.Box 390,
180 Park Avenue
Florham Park, New Jersey 07932-0390(US)**

(72) Inventor: **Iglesia, Enrique
3 Spruce Run Road
Clinton New Jersey 08809(US)**
Inventor: **Madon, Rostam Jal
9 Hendrick Road
Flemington New Jersey 08822(US)**

(74) Representative: **Somers, Harold Arnold et al
ESSO Engineering (Europe) Ltd.
Patents & Licences
Mailpoint 72
Esso House
Ermyn Way
Leatherhead, Surrey KT22 8XE (GB)**

**Description**

The present invention relates to a process for reducing methane production and for increasing liquid yields in Fischer-Tropsch reactions.

U.S. patent US-A-4547525 discloses a process for reducing methane production in Fischer-Tropsch processes wherein hydrocarbons are synthesized from feeds comprising mixtures of CO and $H_2$ by adding at least one olefin to the feed. In a preferred embodiment, the olefin or olefins will comprise one or more $C_2$-$C_{10}$ alpha olefins with an olefin to CO mol ratio of from about 1:10 to 3:4, and the catalyst will comprise at least one Group VIII metal supported on an inorganic refractory oxide support.

The present invention provides a process for reducing methane formation and for increasing liquid ($C_{5+}$) yields in a Fischer-Tropsch process for synthesizing hydrocarbons from CO and $H_2$, comprising adding at least one olefin to a catalyst-containing reactor bed, characterized in that a gas mixture containing CO and $H_2$ is introduced at the inlet end of the reactor bed and the olefin is separately introduced directly into the bed at a position which is more than 10% of the length of the bed from the inlet end of the bed and more than 10% of the length of the bed from the outlet end of the bed.

The amount of said olefin added to said reactor bed may be based on an olefin to CO mole ratio in the range of from 1:100 to 5:1 (e.g., in the range of from 1:20 to 5:1).

The said olefin(s) may be alpha olefin of the type $R\text{-}CH=CH_2$, wherein R is hydrogen or an alkyl group having 1 to 17 carbon atoms. The said olefin may comprise a $C_2$-$C_{10}$ alpha olefin.

The catalyst may comprise one or more Group VIII catalytic metals. The catalytic metal may be selected from iron, cobalt and ruthenium.

The process may be performed at a temperature of at least 100°C, preferably in the range of from 100°C to 500°C.

Water may be added to the reactor bed at a position which is more than 10% of the length of the bed from the inlet end of the bed and more than 10% of the length of the bed from the outlet end of the bed.

The molar ratio of water to CO may be in the range of from 0.1:1 to 5.0:1. The mol ratio of water to olefin may be in the range of from 0.1:1 to 50:1 (e.g., in the range of from 0.2:1 to 10:1).

The present invention is based on the discovery that liquid ($C_{5+}$) yields and methane production in Fischer-Tropsch hydrocarbon synthesis reactions are respectively increased and reduced by adding one or more olefins directly into the reactor bed or by adding water and one or more olefins directly into the reactor bed in a range of positions in the bed in accordance with the invention as specified herein. Thus, the instant invention relates to a process for reducing methane production in Fischer-Tropsch processes wherein hydrocarbons are synthesized from feeds comprising mixtures of CO and $H_2$ and by adding at least one olefin to the reactor bed or water and one or more olefins, wherein the olefin may be obtained by separation from the product stream and recycled to the reactor bed or from an independent source, such as processing the paraffinic product of the Fischer-Tropsch reaction through a dehydrogenation reactor to convert the paraffins into olefins. In a preferred embodiment, the olefin or olefins will comprise one or more $C_2$-$C_{20}$ alpha olefins, wherein the olefin to CO mole ratio in the reactor is from 1/20 to 5/1, more preferably from 1/10 to 2/1, and still more preferably from about 1/5 to 1/1. The molar ratio of water to olefin may be 0.1 to 50, more preferably 0.2 to 10 and most preferably 0.5 to 10. The catalyst will comprise at least one Group VIII metal supported on an inorganic refractory oxide support. Alternatively, cobalt or iron catalyst can be employed.
In a particularly preferred embodiment the catalyst will comprise ruthenium supported on titania.

DETAILED DESCRIPTION OF THE INVENTION

It has now been discovered that methane production in Fischer-Tropsch hydrocarbon synthesis reactions is reduced by adding one or more olefins directly into the reactor bed or by adding water and one or more olefins directly into the reactor bed at a position or positions which is or are more than 10% of the length of the bed from the inlet end of the bed and more than 10% of the length of the bed from the outlet end of the bed. Thus, the instant invention relates to a process for reducing methane production in Fischer-Tropsch processes wherein hydrocarbons are synthesized from feeds comprising mixtures of CO and $H_2$ and by adding at least one olefin and or water and one or more olefin to the reactor bed, wherein the olefin is obtained by separation from the product stream and recycled to the reactor bed or from an independent source. In a preferred embodiment, the olefin or olefins will comprise one or more $C_2$-$C_{20}$ alpha olefins, wherein the olefin to CO mole ratio in the reactor is from 1/20 to 5/1, more preferably from 1/10 to 2/1, and most preferably from 1/5 to 1/1. The molar ratio of water to olefin is 0.1 to 50, more preferably 0.2 to 10 and most preferably 0.5 to 10. The catalyst will comprise at least one Group VIII metal supported on an inorganic refractory oxide support. In a particularly preferred embodiment the catalyst will comprise ruthenium supported on titania. Additionally, cobalt or iron catalyst can be readily employed.

Preferred olefins useful in the process of the instant invention include alpha olefins of the type R-$CH=CH_2$ wherein R is hydrogen or an alkyl group having 1 to 17 carbon atoms, more preferably the alkyl group has 1 to 11 carbon atoms, and most preferably the alkyl group has 1 to 8 carbon atoms. Still more preferably are $C_2$-$C_{10}$ alpha olefins. The amount of alpha olefin recycled to the reactor bed will be sufficient to maintain an olefin to CO mole ratio of from 1/100 to 5/1, more preferably 1/20 to 5/1, and most preferably 1/10 to 2/1. Also, internal olefins where unsaturation is away from the terminal carbon can also be added beneficially to the $CO/H_2$ feed.

Although the process of the instant invention may be practiced in the presence of any suitable Fischer-Tropsch catalyst, in a preferred embodiment it will be practiced in the presence of a catalyst comprising one or more Group VIII metals supported on an inorganic refractory oxide support, preferably ruthenium or cobalt supported on such a support. Thus, suitable supports include oxides of titanium (e.g. titania), niobium (e.g niobia), vanadium, tantalum, silicon (i.e., silica), aluminium (i.e., alumina), manganese and mixtures thereof. Preferably the catalyst support will be selected from the group consisting of titania, zirconium titanate, mixtures of titania and alumina, mixtures of titania and silica, alkaline earth titanates, alkali titanates, rare earth titanates and mixtures of any one of the foregoing with supports selected from the group consisting of vanadia, niobia, tantala, alumina, silica and mixtures thereof. Thus, in a particularly preferred embodiment of this invention the process will be carried out in the presence of a catalyst comprising ruthenium supported on a titania support. Alternatively, the catalyst can comprise cobalt or iron supported on one of the aforementioned inorganic refractory oxides.

In general, the amount of cobalt catalytic metal present is 1 to 50 weight percent of the total catalyst composition, more preferably from 10.0 to 25 weight percent.

Iron catalysts containing 10 to 60 weight percent iron, more preferably 20 to 60 weight percent, and most preferably 30 to 50 weight percent, are unsupported, but promoted with refractory metal oxide ($SiO_2$, $Al_2O_3$, etc.), alkali (K, Na, Rb) and Group IB metal (Cu, Ag). These catalysts are usually calcined, but usually not reduced, rather they are brought up to reaction temperature directly in the $CO/H_2$ feed.

In general, the amount of ruthenium catalytic metal present on the catalyst will generally range from 0.01 to 50 weight percent of the total catalyst composition, more preferably from 0.1 to 5.0 weight percent and most preferably from about 0.5 to about 5 weight percent.

In general, in the process of this invention the Fischer-Tropsch reactor temperature will broadly range from 100°C to 500°C, more preferably from 150°C to 300°C and most preferably 200°C to 270°C, at a pressure of 100 to 10,000 kPa, more preferably 300 to 5,000 kPa and most preferably 500 to 3,000 kPa. The space velocity ($V_1$) of the feed gas ($H_2 + CO$) will range from 10 to 10,000 standard $cm^3/hr$-($cm^3$ of catalyst), more preferably 100-4,000 and most preferably 200 to 2,000. The space velocity ($V_2$) of the alpha olefin will range from 0.1 to about 20,000 standard $cm^3/hr$-($cm^3$ of catalyst), more preferably 3-4,000 and most preferably 10 to 1,000. Thus, the volume ratio of $V_1/V_2$ is 0.005 to 10,000, more preferably 0.02 to 1,000 and most preferably 0.5 to 200. The hydrogen to carbon monoxide mole ratio of the feed gas, $H_2/CO$, will range from 0.5 to 10 and preferably from 1 to 3.

If the alpha olefin is used without water addition, the alpha olefin is injected into the reactor through an inlet port in the sidewall or center of the reactor, wherein the port is positioned below a point which is 10% of the distance from the top of the reactor bed to the bottom of the reactor bed and above a point which is 10% of the distance from the bottom of the reactor bed to the top of the reactor bed.

Water may be injected into the top of the reactor through inlet ports in the sidewall of the reactor or through inlet ports which are positioned anywhere in the reactor below a point which is 10% of the distance from the top of the reactor bed to the bottom of the reactor bed and above a point which is 10% of the distance from the bottom of the reactor bed to the top of the reactor bed. The ports for the water and the ports for the alpha olefin can be located at different points within the reactor.

The reactor bed can comprise a single reactor bed of one catalyst system or it can comprise two catalyst beds with different Fischer-Tropsch synthesis catalysts separated by an inert inter-stage knock-out zone. The water and/or alpha olefin can also be injected through an inlet port located at the interstage knock-out zone. The alpha olefin can also be injected through an inlet port located at the inter-stage knock-out zone. Alternatively, the reactor bed can comprise a plurality of tubular reactors joined in a serial fluid communication wherein the alpha olefin is injected into the reactor bed in accordance with the invention either within the first tubular reactor or subsequent thereto with or without inter-stage knock-out zone.

3

## DESCRIPTION OF THE PREFERRED EMBODIMENTS

The invention will be more readily understood by reference to the following Examples.

Fischer-Tropsch Hydrocarbon Synthesis

Experimental Procedure

Anywhere from about 5 to 40 g of catalyst was loaded into a stainless steel reactor with an ID of 0.78 cm, having a thermocouple placed within the catalyst bed to measure temperature. The rector was flushed with He at a flow rate of 500 $cm^3$/min. and then pressurized (in He) at 30 atmospheres to confirm the integrity of the unit. The He in the reactor was then brought back to 1 atmosphere and replaced with $H_2$ at a space velocity between 200-600 v/v/hr at room temperature. The temperature was raised in approximately 2 to 3 hours to a final reduction temperature of between 400°C to 500°C and held at this temperature overnight.

After reduction, each catalyst was brought on stream in either one of two ways, which were found to be substantially equivalent. In one method, the reduced catalyst was treated for 15 to 50 hours at 1 atmosphere $2H_2$/CO at 200°C (the space velocity of the $H_2$/CO used was varied from 200-1,000 $cm^3 H_2$ + CO h. (cc catalyst). The conditions in the reactor were then adjusted to the desired temperature, pressure and space velocity. In the other method, the atmospheric treatment in $H_2$/CO was not done and the catalysts were brought on-stream at the desired experimental pressure in $H_2$/CO, but an initial low temperature of 150-170°C, which was then slowly raised (in 1 to 5 hours) to the desired experimental temperature.

## EXAMPLES

In all of the Examples, the determination of the Fischer-Tropsch synthesis activity towards the various products was measured by employing an on-line gas chromatograph using an isothermal, fixed-bed, stainless steel reactor (0.78 cm ID) into which from about 5 to 40 g of catalyst was loaded. A thermocouple was placed within the catalyst bed to measure the reaction temperature.

### Example 1

#### Preparation of Ru Catalyst

Titanium dioxide supports prepared by pressing Degussa P-25 powder (20-70% rutile) into wafers using a hydraulic press at a pressure of about 20,000 lb/$in^2$. The wafers were then crushed in a mortar to a coarse powder and sieved to retain the 80-140 mesh (U.S. standard) fraction for all the $TiO_2$ supported catalysts. The so-formed titanium dioxide support samples were calcined in flowing air overnight (about 1 liter per minute) at a temperature of 550°C to 600°C. The calcined samples were then reduced in hydrogen flowing at a rate of 1 liter per minute by heating from room temperature to 450°C for 4 hours, after which the sample was cooled in flowing $H_2$, flushed with He and discharged. These reduced samples possessed the characteristic blue-gray color of surface-reduced $TiO_2$.

Catalysts were produced by impregnating the support material with a ruthenium nitrate/acetone mixture. The nitrate was obtained from Engelhardt as 10% weight ruthenium metal in nitric acid.

The nitrate-impregnated $TiO_2$ was reduced in flowing hydrogen (1 1/min.) as follows: (a) heated from room temperature to 100°C and held for 0.5 hours; (b) heated from 100°C to 450°C at 3°C/min. and held for 4 hours; (c) cooled in $H_2$ to room temperature overnight; and (d) flushed in the reduction cell with He or Ar (1 1/min.) for one hour. Each sample of reduced catalyst was then passivated by introducing 1% $O_2$ into the He stream and then increasing the $O_2$ content to 100% over a period of from about 2-3 hours.

### Example 2

#### Preparation of Cobalt Catalyst

Degussa P-25 $TiO_2$ was treated with acetone, dried at 90°C and mulled with 9.6 weight percent Sterotex (vegetable stearine). This material was tableted to 3/8 inch size, using a pilling pressure which yielded a material of 0.35-0.40 ml/g pore volume. The 3/8 inch pills were crushed and screened to 42-80 mesh size and calcined in flowing air at 500°C for 4 hours to burn out the sterotex. Portions were then re-calcined in flowing air at 650°C for 16 hours to obtain a high rutile content and screened to 80-150 mesh size. The support composite used to prepare this catalyst was 97% rutile, 13.6 $m^2$/g, 0.173 ml/g.

Impregnation Procedure

108.8 g cobaltous nitrate ($Co(NO_2)_2$ . $6H_2O$ and 16 ml aqueous perrhenic acid solution (52 mg Re/ml) were then dissolved in about 300 ml acetone in a 1 liter round bottom flask. 160 g $TiO_2$ (YAX-0487) was added and the mixture placed on a rotary evaporator until dry. The catalyst was dried in a vacuum oven at 140°C for 18 hours and calcined in flowing air at 250°C for 3 hours. The calcined catalyst was re-screened to remove the small amount of fines generated during preparation.

Cobalt Catalyst Preparation

Analytical inspections for the catalyst are given below:

| | |
|---|---|
| Cobalt, Wt. % | 11.3 |
| Rhenium, Wt. % | 0.42 |
| Sodium ppm | 369 |
| % Rutile (ASTM D 3720-78) | 97 |
| Surface Area, $m^2$/g | 14.6 |
| Pore Volume, ml/g | 0.122 |
| Bulk Density, g/ml | 1.41 |

Besides quantifying rutile content, the x-ray diffraction spectrum indicates the presence of $CO_3O_4$.

Example 3 (This is not an example according to the invention)

Effect of Ethylene Feed-Cobalt Catalysts

The general procedure described for Fischer-Tropsch hydrocarbon synthesis was modified as follows in Examples 3 and 4.

The catalyst was started in $H_2$/CO at a space velocity required to achieve 25-45% CO conversion and at 1,700-2,500 Kpa. An ethylene/argon mixture was mixed with $H_2$/CO feed and then introduced to reactor at inlet at an amount of ethylene to give 2.5% and 6.2% ethylene in feed on Co and Ru catalysts, respectively. The total reactor pressure was then increased to the level necessary to maintain the $H_2$ + CO partial pressure in the reactor at the level found before ethylene addition. The reactor was operated at these conditions for 4 to 24 hours. Ethylene/argon feed was then directed so that it entered the reactor, undiluted by $H_2$ + CO, at a point in the reactor 1/3 x L (L reactor length) down from inlet, while maintaining $H_2$ + CO space velocity and partial pressure at its previous level. The reactor was operated at these conditions for 4 to 24 hours. The ethylene/argon feed was discontinued and reactor conditions returned to those of beginning in order to rule out irreversible deleterious effects of ethylene addition on synthesis activity and selectivity.

| Run | 119-342 | 119-339 |
|---|---|---|
| % Ethylene in Feed | 0 | 2.5 |
| Location Added | None | Inlet |

Ethylene

| % Ethylene Converted | - | 99.5 |
|---|---|---|
| Selectivity (%)* to | | |
| $C_2H_6$ | - | 86.5 |
| $C_3^+$ | - | 13.5 |
| $C_3^+$ Yield (%)** | - | 13.5 |

| Selectivity (%)*** | | |
|---|---|---|
| $CH_4$ | 9.4 | 8.3 |
| $C_2$ | 1.3 | - |
| $C_3$ | 2.7 | 3.3 |
| $C_4$ | 2.4 | 2.9 |
| $CO_2$ | 0.4 | 0.5 |
| $C_5^+$ | 83.8 | 92.9 |

\* % of ethylene <u>converted</u> which appeared as these product.

\*\* % of ethylene <u>added</u> which appeared as these products.

\*\*\* Selectivity is defined as percent of the CO converted which appears as a given product (it is almost identical to weight percent). However, when ethylene is added some of the C-atoms come from it and the sum of selectivities is greater than 100%.

11.7% $Co/0.5$ $Re/TiO_2$, 200°C, 2,050 kPa, $H_2$ + CO, $H_2/CO$ = 2.1/1
25-27% CO conversion
The addition of ethylene ($C_2^=$) at reactor inlet lowers the $CH_4$ selectivity and increases $C_5^+$ selectivity. Most of the ethylene (at 2.5% concentration) is converted, but only 13.5% of the converted ethylene

appears as desirable $C_3^+$ products; the rest (86.5%) is hydrogenated to ethane, which is unreactive towards further chain growth to $C_3^+$ at Fischer-Tropsch synthesis conditions and, thus, cannot be recycled without converting it to ethylene in a dehydrogenation reactor.

Example 4 (This is not an example according to the invention).

Effect of Ethylene Feed-Ruthenium Catalysts

| Run | 121-455 | 121-454 |
|---|---|---|
| % Ethylene in Feed | 0 | 6.2 |
| Location Added | None | Inlet |

**Ethylene**

| % Ethylene Converted | - | 97 |
|---|---|---|
| Selectivity to (%)* | | |
| Ethane | - | 82 |
| $C_3^+$ | - | 18 |
| Yield (%) $C_3^{+**}$ | - | 17 |
| Selectivity (%)*** | | |
| $CH_4$ | 5.5 | 4.3 |
| $C_2$ | 0.7 | - |
| $C_3$ | 2.1 | 2.6 |
| $C_4$ | 3.5 | 4.0 |
| $CO_2$ | 0.7 | 0.7 |
| $C_5^+$ | 87.5 | 105.6 |

\*    % of ethylene converted which appeared as these products.

\*\*    % of ethylene added which appeared as these products.

\*\*\*    Selectivity is defined as percent of the CO converted which appears as a given product (it

is almost identical to weight percent). How-
ever, when ethylene is added some of the C-
atoms come from it and the sum of selectivities
is greater than 100%.

1.2% Ru/TiO$_2$, 200°C, 1,700 kPa, H$_2$/CO = 2.1/1, 35-45% CO conversion.

Addition of ethylene to H$_2$/CO feed at reactor inlet (at 6.2% level) lowers the CH$_4$ selectivity from 5.5 to 4.3, while increasing the C$_5^+$ selectivity from 87.5 to 105.6%. Most of the ethylene added in (97%) is converted, of which only 18% appears as desirable C$_3^+$ products; the rest (82%) is hydrogenated to ethane, which is unreactive towards further chain growth to C$_3^+$ at Fischer-Tropsch synthesis conditions and, thus, cannot be recycled without converting it to ethylene in a dehydrogenation reactor.

Example 5

Top versus Below Inlet Feed Ethylene-Ru

There is a clear advantage to bypassing the reactor inlet by introducing the C$_2^=$ below the inlet at the same concentration. The CH$_4$ selectivity decreases further (from 4.3% to 3.9%), the C$_5^+$ selectivity increases (from 105.6% to 117.0%) and the activity of the catalyst is unaffected by the presence of ethylene, either at inlet or below the inlet of the reactor. The apparent reason for the improved product selectivity is an increase in the yield of C$_3^+$ from ethylene (the percentage of the ethylene feed converted to C$_3^+$) from 17% to 26% in spite of the lower ethylene conversion when added below the inlet. Below inlet addition dramatically decreases the hydrogenation selectivity (from 82% to 44%), while increasing the C$_3^+$ selectivity (from 18% to 56%). Therefore, it dramatically increases the efficiency of ethylene utilization while avoiding the formation of species such as ethane which cannot be polymerized further under the Fischer-Tropsch conditions.

| Run | 121-453 | 121-454 |
|---|---|---|
| % Ethylene in Feed | 6.2 | 6.2 |
| Location Added | Below Top Third of Reactor | Top |

Ethylene*

| % Ethylene Converted | 45.3 | 97 |
|---|---|---|
| Selectivity to (%) | | |
| Ethane | 44 | 82 |
| $C_3^+$ | 56 | 18 |
| Yield (%) $C_3^{+**}$ | 26 | 17 |
| Selectivity (%)*** | | |
| $CH_4$ | 3.9 | 4.3 |
| $C_2$ | - | - |
| $C_3$ | 3.4 | 2.6 |
| $C_4$ | 5.1 | 4.0 |
| $CO_2$ | 0.7 | 0.7 |
| $C_5^+$ | 117.0 | 105.6 |

\* % of ethylene converted which appeared as these products.

\*\* % of ethylene added which appeared as these products.

\*\*\* Selectivity is defined as percent of the CO converted which appears as a given product (it is almost identical to weight percent). However, when ethylene is added some of the C-atoms come from it and the sum of selectivities is greater than 100%.

1.2% Ru/TiO$_2$, 200 °C, 1,700 kPa, H$_2$/CO = 2.1/1, 35-45% CO conversion.

Example 6

Top versus Below Inlet Feed Ethylene-Co

There is a clear advantage to bypassing the reactor inlet by introducing the $C_2^=$ below the inlet at the same concentration. The $CH_4$ selectivity decreases further (from 8.3% to 7.2%), the $C_5^+$ selectivity increases (from 92.9% to 99.3%) and the activity of the catalyst is unaffected by the presence of ethylene, either at inlet or below the inlet of the reactor. The apparent reason is an increase in the yield of $C_3^+$ from ethylene (the percentage of the ethylene feed converted to $C_3^+$) (from 13.% to 27.3%), in spite of the lower ethylene conversion when added below the inlet. Below inlet addition dramatically decreases the hydrogenation selectivity (from 86.5% to 69.5%), while increasing the $C_3$ selectivity (from 13.5% to 30.5%). Therefore, it dramatically increases the efficiency of ethylene utilization, while avoiding the formation of species such as ethane which cannot be polymerized under Fischer-Tropsch conditions.

| Run | 119-336 | 119-339 |
|---|---|---|
| % Ethylene in Feed | 2.5 | 2.5 |
| Location Added | Below Top Third | Inlet |
| **Ethylene** | | |
| % Ethylene Converted | 89.5 | 99.5 |
| Selectivity (%)* to | | |
| $C_2H_6$ | 69.5 | 86.5 |
| $C_3^+$ | 30.5 | 13.5 |
| $C_3^+$ Yield (%)** | 27.3 | 13.5 |
| Selectivity (%) *** | | |
| $CH_4$ | 7.2 | 8.3 |
| $C_2$ | – | – |
| $C_3$ | 3.7 | 3.3 |
| $C_4$ | 3.7 | 2.9 |
| $CO_2$ | 0.4 | 0.5 |
| $C_5^+$ | 99.3 | 92.9 |

\* % of ethylene <u>converted</u> which appeared as these products.

\*\* % of ethylene <u>added</u> which appeared as these products.

   *** Selectivity is defined as percent of the CO con-
verted which appears as a given product (it is
almost identical to weight percent).  However,
when ethylene is added some of the C-atoms come
from it and the sum of selectivities is greater
than 100%.

11.7% Co/0.5 Re/TiO$_2$, 200°C, 2,050 kPa, H$_2$ + Co, H$_2$/CO - 2.1/1
25-27% CO conversion.

Example 7 (This is not an example according to the invention)

Experimental Procedure for Ethylene Addition

The catalyst was started in H$_2$/CO at a space velocity required to achieve 15-30% CO conversion at 2,000-2,100 kPa. Conditions were maintained for at least 48 hours. An ethylene/argon mixture was then mixed with the CO/H$_2$ feed and introduced to the reactor at the inlet at an ethylene concentration of 2-7% by volume. The total reactor pressure was then increased so as to maintain H$_2$ + CO partial pressure equal to that prior to ethylene cofeed. The reactor was operated at these conditions for at least 24 hours.

Effect of Ethylene Addition - Ruthenium Catalyst

| Run | 123-608 | 123-609 |
| --- | --- | --- |
| % Ethylene in Feed | 0 | 7 |
| % Ethylene Converted | - | 73.4 |
| Selectivity to (%)* | - | |

| | | |
|---|---|---|
| Ethane | – | 50.5 |
| $C_3^+$ | – | 49.5 |

Yield to (%)**

| | | |
|---|---|---|
| $C_3^+$ | – | 36.3 |

Rate of Ethylene Conversion

| (mol $C_2^=$/g. atom Ru.h) $C_3^+$ | – | 6.1 |
|---|---|---|
| $C_2H_6$ | – | 6.3 |

Rate of CO conversion

| (mol CO/g.atom Ru.H) | 12.8 | 12.5 |
|---|---|---|

CO Selectivity (%)***

| | | |
|---|---|---|
| $CH_4$ | 3.7 | 3.0 |
| $C_2$ | 0.4 | – |
| $C_3$ | 1.75 | 2.26 |
| $C_4$ | 2.89 | 3.4 |
| $CO_2$ | 0.3 | 0.4 |
| $C_5^+$ | 91.0 | 188.5 |

1.2% $Ru/TiO_2$, 191-192$^o$C, 2,100 kPa, $H_2$+CO, $H_2$/CO = 2.1/1, 18-22% CO conversion.

\* % of ethylene converted which appeared as these products.

\*\* % of ethylene added which appeared as these products.

\*\* Selectivity is defined here as the percent of CO converted which appears as a given product. However, when ethylene is added some of the C-atoms come from it and the value of this "selectivities" can exceed 100%.

14

The addition of ethylene ($C_2^=$) to the $H_2$/CO feed at reactor inlet lowers the $CH_4$ selectivity and increases the $C_5^+$ selectivity on the Ru/$TiO_2$ catalyst. A large fraction of the ethylene (7% concentration to the feed) added is converted (73.4%), but only 49.5% of the converted $C_2^=$ appears as desired $C_3^+$ products; the rest (50.5%) is hydrogenated to ethane, which is unreactive towards further chain growth at Fischer-Tropsch synthesis conditions and, thus, cannot be recycled without converting it to ethylene in a dehydrogenation reactor.

Example 8 (This is not an example according to this invention)

Experimental Procedure for Ethylene Addition and Water Co-feed

The catalyst was started in $H_2$/CO at a space velocity required to achieve 15-30% CO conversion at 2,000-2,100 kPa. Conditions were maintained for at least 48 hours. An ethylene/argon mixture was then mixed with the CO/$H_2$ feed and introduced to the reactor at the inlet at an ethylene concentration of 2-7% by volume. The total reactor pressure was then increased so as to maintain $H_2$ + CO partial pressure equal to that prior to ethylene cofeed. The reactor was operated at these conditions for at least 24 hours. When water vapor was introduced at a concentration of 10-20 volume percent by bubbling the $H_2$ + CO feed through a saturator maintained at 120-145°C, while keeping the ethylene/argon flow constant. The total reactor pressure was increased so as to maintain the $H_2$ + CO and the ethylene partial pressures at their level prior to water cofeed. Also, the flow rates of $H_2$/CO and of ethylene/argon were increased so as to maintain the CO conversion at their level prior to water addition. The reactor was operated at these conditions for at least 24 hours.

15

Effect of Ethylene/Water Addition - Ruthenium Catalyst

| Run | 123-609 | 123-614 |
|---|---|---|
| % Ethylene in Feed | 7 | 7 |
| % Water in Feed | 0 | 18 |
| % Ethylene Converted | 73.4 | 38.2 |
| Selectivity to (%) * | | |
| Ethane | 50.5 | 11.5 |
| $C_3^+$ | 49.5 | 88.5 |
| Yield (%)** | | |
| $C_3^+$ | 36.3 | 33.8 |
| Rate of Ethylene Conversion to (mole $C_2^=$/g.atom Ru.h) | | |
| $C_3^+$ | 6.1 | 17.3 |
| $C_2H_6$ | 6.3 | 2.2 |

Rate of CO Conversion

(mol CO/g.atom Ru.h)          12.5          34.0

CO Selectivity (%)***

$CH_4$          3.0          0.9

$C_2$          -          -

$C_3$          2.26          0.97

$C_4$          3.40          1.46

$CO_2$          0.4          0.4

$C_5^+$          188.5          198.5

1.2% $Ru/TiO_2$, 191-192°C, 2,100 kPa, $H_2+CO$, $H_2/CO$ = 2.1/1, 18-22% CO conversion.

\* % of ethylene converted which appeared as these products.

\*\* % of ethylene added which appeared as these products.

\*\*\* Selectivity is defined here as the percent of CO converted which appears as a given product. However, when ethylene is added some of the C-atoms come from it and the value of this "selectivities" can exceed 100%.

The Table shows that there is a clear advantage to the cofeeding of water vapor with the ethylene and with the $H_2/CO$ feed. While the ethylene conversion decreases from 73.5% to 38.2% with the addition of 18% $H_2O$, the yield decreases only slightly (36.3% to 33.8%). However, most importantly:

o the selectivity to desired $C_3^+$ products increases from 49.5% to 88.5%;

o the rate of ethylene conversion to $C_3^+$ products and of CO conversion increase approximately threefold; in other words, the activity of the catalyst for both reactions, $C_2H_4$ incorporation and Fischer-Tropsch synthesis, increases threefold.

o the rate of ethylene conversion to ethane (i.e., hydrogenation rate), actually decreases approximately threefold with the addition of 18% water vapor to the $H_2/CO/C_2^=$ feed.

Accompanying beneficial effects are a decrease in the $CH_4$ selectivity (3.0% to 0.9%) and an increase in the effective $C_5^+$ selectivity from 138.5% to 199.5%.

These results suggest that the effect of water is to enhance the reincorporation and chain growth of added ethylene while dramatically inhibiting its hydrogenation to undesired ethane product.

Notes

   o  Mass expressed in pounds (lbs) is converted to kg equivalent by multiplying by 0.4536.
   o  Length expressed in inch is converted to cm by multiplying by 2.54.

**Claims**

1.  A process for reducing methane formation and for increasing liquid ($C_{5+}$) yields in a Fischer-Tropsch process for synthesizing hydrocarbons from CO and $H_2$, comprising adding at least one olefin to a catalyst-containing reactor bed, characterized in that a gas mixture containing CO and $H_2$ is introduced at the inlet end of the reactor bed and the olefin is separately introduced directly into the bed at a position which is more than 10% of the length of the bed from the inlet end of the bed and more than 10% of the length of the bed from the outlet end of the bed.

2.  The process of claim 1 wherein the amount of said olefin added to said reactor bed is based on an olefin to CO mole ratio in the range of from 1:100 to 5:1.

3.  The process of claim 2 wherein the olefin:CO mole ratio is in the range of from 1:20 to 5:1.

4.  The process of any one of claims 1 to 3 wherein said olefin(s) is or are alpha olefin of the type R-$CH = CH_2$, wherein R is hydrogen or an alkyl group having 1 to 17 carbon atoms.

5.  The process of any one of claims 1 to 4 wherein said olefin comprises a $C_2$-$C_{10}$ alpha olefin.

6.  The process of any one of claims 1 to 5 wherein said catalyst comprises one or more Group VIII catalytic metals.

7.  The process of claim 6 wherein said catalytic metal is selected from iron, cobalt and ruthenium.

8.  The process of any one of claims 1 to 6 performed at a temperature of at least 100°C.

9.  The process of claim 8 performed at a temperature in the range of from 100°C to 500°C.

10. The process of any one of claims 1 to 9 comprising adding water to the reactor bed at a position which is more than 10% of the length of the bed from the inlet end of the bed and more than 10% of the length of the bed from the outlet end of the bed.

11. The process of claim 10 wherein the molar ratio of water to CO is in the range of from 0.1:1 to 5.0:1.

12. The process of claim 10 or claim 11 wherein the mol ratio of water to olefin is in the range of from 0.1:1 to 50:1.

13. The process of claim 12 wherein the mol ratio of water to olefin is in the range of from 0.2:1 to 10:1.

**Patentansprüche**

1.  Verfahren zur Verringerung der Methanbildung und zur Erhöhung der Ausbeuten an $C_{5+}$-Flüssigkeiten in einem Fischer-Tropsch-Verfahren zum Synthetisieren von Kohlenwasserstoffen aus CO und $H_2$, bei dem einem katalysatorhaltigen Reaktorbett mindestens ein Olefin zugesetzt wird, dadurch gekennzeichnet, daß eine Gasmischung, die CO und $H_2$ enthält, an dem Eintrittsende des Reaktorbettes eingebracht wird und das Olefin getrennt davon direkt in das Bett an einer Stelle eingebracht wird, die mehr als 10 % der Länge des Bettes von dem Eintrittsende des Bettes und mehr als 10 % der Länge des Bettes von dem Austrittsende des Bettes entfernt ist.

2.  Verfahren nach Anspruch 1, bei dem die Menge des dem Reaktorbett zugesetzten Olefins auf einem Molverhältnis von Olefin zu CO im Bereich von 1:100 bis 5:1 basiert.

3.  Verfahren nach Anspruch 2, bei dem das Olefin:CO-Molverhältnis im Bereich von 1:20 bis 5:1 liegt.

18

4. Verfahren nach einem der Ansprüche 1 bis 3, bei der das Olefin bzw. die Olefine α-Olefin des Typs R-CH=CH$_2$ ist bzw. sind, wobei R Wasserstoff oder eine Alkylgruppe mit 1 bis 17 Kohlenstoffatomen ist.

5. Verfahren nach einem der Ansprüche 1 bis 4, bei dem das Olefin ein C$_2$- bis C$_{10}$-α-Olefin umfaßt.

6. Verfahren nach einem der Ansprüche 1 bis 5, bei dem der Katalysator ein oder mehrere katalytische Gruppe-VIII-Metalle umfaßt.

7. Verfahren nach Anspruch 6, bei dem das katalytische Metall ausgewählt ist aus Eisen, Kobalt und Ruthenium.

8. Verfahren nach einem der Ansprüche 1 bis 6, das bei einer Temperatur von mindestens 100 °C durchgeführt wird.

9. Verfahren nach Anspruch 8, das bei einer Temperatur im Bereich von 100 °C bis 500 °C durchgeführt wird.

10. Verfahren nach einem der Ansprüche 1 bis 9, bei dem dem Reaktorbett an einer Stelle, die mehr als 10 % der Länge des Bettes von dem Eintrittsende des Bettes und mehr als 10 % der Länge des Bettes von dem Austrittsende des Bettes entfernt ist, Wasser zugesetzt wird.

11. Verfahren nach Anspruch 10, bei dem das Molverhältnis von Wasser zu CO im Bereich von 0,1:1 bis 5,0:1 liegt.

12. Verfahren nach Anspruch 10 oder Anspruch 11, bei dem das Molverhältnis von Wasser zu Olefin im Bereich von 0,1:1 bis 50:1 liegt.

13. Verfahren nach Anspruch 12, bei dem das Molverhältnis von Wasser zu Olefin im Bereich von 0,2:1 bis 10:1 liegt.

**Revendications**

1. Procédé de réduction de la formation de méthane et d'augmentation des rendements en C$_{5+}$ liquides dans un procédé de Fischer-Tropsch pour synthétiser des hydrocarbures à partir de CO et de H$_2$, ce procédé consistant à ajouter au moins une oléfine à un lit de réacteur contenant un catalyseur, caractérisé en ce qu'un mélange gazeux contenant du CO et du H$_2$ est introduit à l'extrémité d'entrée du lit du réacteur et l'oléfine est introduite séparément directement dans le lit dans une position qui se trouve à plus de 10 % de la longueur du lit à partir de l'extrémité d'entrée du lit et à plus de 10 % de la longueur du lit à partir de l'extrémité de sortie du lit.

2. Procédé selon la revendication 1, dans lequel la quantité de ladite oléfine ajoutée audit lit de réacteur est basée sur un rapport molaire de l'oléfine au CO dans la plage de 1:100 à 5:1.

3. Procédé selon la revendication 2, dans lequel le rapport molaire de l'oléfine au CO se situe dans la plage de 1:20 à 5:1.

4. Procédé selon l'une quelconque des revendications 1 à 3, dans lequel la ou lesdites oléfines est ou sont des alpha oléfines de type R-CH=CH$_2$, dans lequel R est de l'hydrogène ou un groupe alkyle ayant 1 à 17 atomes de carbone.

5. Procédé selon l'une quelconque des revendications 1 à 4, dans lequel ladite oléfine comprend une alpha oléfine en C$_2$-C$_{10}$.

6. Procédé selon l'une quelconque des revendications 1 à 5, dans lequel ledit catalyseur comprend un ou plusieurs métaux catalytiques du groupe VIII.

7. Procédé selon la revendication 6, dans lequel ledit métal catalytique est choisi parmi le fer, le cobalt et le ruthénium.

**8.** Procédé selon l'une quelconque des revendications 1 à 6 réalisé à une température d'au moins 100°C.

**9.** Procédé selon la revendication 8 réalisé à une température dans la plage de 100 à 500°C.

**10.** Procédé selon l'une quelconque des revendications 1 à 9 dans lequel on ajoute de l'eau au lit du réacteur dans une position qui se trouve à plus de 10 % de la longueur du lit à partir de l'extrémité d'entrée du lit et à plus de 10 % de la longueur du lit à partir de l'extrémité de sortie du lit.

**11.** Procédé selon la revendication 10, dans lequel le rapport molaire de l'eau au CO se situe dans la plage de 0,1:1 à 5,0:1.

**12.** Procédé selon la revendication 10 ou 11, dans lequel le rapport molaire de l'eau à l'oléfine se situe dans la plage de 0,1:1 à 50:1.

**13.** Procédé selon la revendication 12, dans lequel le rapport molaire de l'eau à l'oléfine se situe dans la plage de 0,2:1 à 10:1.